# EUROPEAN PATENT APPLICATION

(11) **EP 4 803 628 A1**
(43) Date of publication of application: **09.09.2026**
(21) Application number: 25382201.9
(22) Date of filing: 05.03.2025
(51) Int. Cl.: C12N 15/115, C12N 15/113

(54) **APTAMERS FOR NUCLEAR TRANSLOCATION**

(71) Applicant: Tyris Therapeutics, S.L., 46980 Paterna (ES)
(72) Inventor: FORTES ALONSO, María Purificación, 31008 PAMPLONA (ES); PASTOR RODRÍGUEZ, Fernando, 31008 PAMPLONA (ES); HERNÁNDEZ SÁEZ, Carlos, 31008 PAMPLONA (ES); OYARZABAL SANTAMARINA, Julen, 46980 PATERNA (ES)
(74) Representative: ZBM Patents - Zea, Barlocci & Markvardsen

(57) **Abstract**

The present invention refers to an oligonucleotide sequences capable of translocating to the cell nucleus. Also provided are conjugates and constructs containing said oligonucleotides for cell delivery, preferably for nuclear cell delivery, and related therapeutic applications.

## Description

### Technical Field

The present invention belongs to the field of genetic engineering and gene therapy. In particular, it refers to new aptamers for nuclear translocation of a cargo to the cell nucleus.

### Background Art

Recently, gene therapy has been developed for the treatment of debilitating human disorders, including diabetes and various cancers. These therapies can overcome the intrinsic disadvantages and serious risks of pharmacological agents. Gene therapy aims to introduce novel genes or to repair malfunctioning genes as a means to permanently treat or reverse the disorders.

In gene therapy, the exogenous "good" DNA is used to replace the defective DNA at appropriate chromosomal targets, correcting mutations or defective genetic functions. However, in practice, the therapeutic genes or constructs encounter various barriers after they are administered in vivo. One of the major hurdles in this process is successful nuclear translocation. Although viral vectors can efficiently infect cells, non-viral vectors are extremely inefficient in terms of the exogenous protein production per copy of therapeutic DNA.

A great research effort has been directed towards facilitating nuclear delivery of therapeutic genes. A common approach is employing nuclear localization signal (NLS) peptides complexed with the nucleic acid construct. It is generally accepted that cargos with classic NLSs are recognized by members of the importin superfamily of cellular nuclear transport proteins and are then translocated into the nucleus. Although several NLSs are known, there are technical and practical difficulties associated to the preparation and use of nucleotide constructs attached to NLS peptides.

Nuclear translocation of nucleic acid constructs has also been attempted by nucleic acid complexing with synthetic lipid nanoparticles to form a structure similar to the plasma membrane, or receptor-targeting nanoparticles for receptor-mediated endocytosis.

Despite the advances in the field, there is a need to develop further efficient strategies for nuclear translocation of nucleic acid constructs, thereby to improve gene therapy efficiency.

### Summary of Invention

The inventors have found new oligonucleotide sequences that effectively translocate to the cell nucleus.

In a first aspect, the invention provides an oligonucleotide comprising: a sequence selected from the group consisting of SEQ ID NO: 1 to SEQ ID NO: 156, or a sequence having at least 80% identity with a sequence selected from the group consisting of SEQ ID NO: 1 to SEQ ID NO: 156 and that is capable of translocating to the cell nucleus.

As shown in the examples below, all oligonucleotides of the first aspect were found in the nuclear fraction of HEK293T cells after incubating said HEK293T cells with the nucleotides and nuclear fractionation. Thus, these oligonucleotides were shown to be capable of translocating from the extracellular medium to the nucleus of HEK293T. Additionally, incubation of HEK293T cells with AF647-labeled oligonucleotides showed higher fluorescence than HEK293T control cells, indicating that the oligonucleotides of the first aspect are properly internalized into cells. Fluorescence microscopy assays further demonstrated that selected florescence-labelled oligonucleotides showed increased nuclear staining after incubation with the HEK293T cells. These results effectively demonstrate that the oligonucleotides of the first aspect translocate to the cell nucleus and may consequently be used for nuclear delivery of a cargo component, such as, for example, a drug, an interfering RNA or a gene of interest.

A second aspect provides a conjugate comprising at least one delivery component and at least one cargo component, wherein the at least one delivery component comprises an oligonucleotide as defined in the first aspect.

When the cargo component is a nucleic acid, the conjugate is generally termed "construct" or "nucleic acid construct". Accordingly, a third aspect of the invention refers to a nucleic acid construct comprising at least one delivery component and at least one cargo component, wherein the at least one delivery component comprises an oligonucleotide as defined in the first aspect and the at least one cargo component is a nucleic acid, such as, for example, a gene, or an interference RNA.

The oligonucleotides, conjugates and constructs of the invention may be formulated in a variety of compositions, including pharmaceutical, cosmetic and diagnostic compositions, with excipients and carriers. Thus, a fourth aspect of the invention relates to a composition comprising the oligonucleotides, conjugates and constructs defined herein together with one or more appropriate excipients or carriers.

A fifth aspect provides a method for delivering a cargo component to a cell, in particular delivering a cargo component to the nucleus of a cell, said method comprising contacting a conjugate or construct as defined in the second or third aspects with the cell. A sixth aspect relates to the use of an oligonucleotide as defined herein for cell-delivery, in particular, nuclear-targeted cell delivery, of a cargo component, i.e. for nuclear-targeted delivery.

The oligonucleotides, conjugates and constructs of the invention may be used in medicinal, cosmetic and diagnostic applications. By effectively delivering a cargo component to the cell nucleus, the oligonucleotides, conjugates and constructs of the invention solve the problems outlined above and advantageously enhance therapeutic approaches that require nuclear delivery of the active agent. Diagnosis and cosmetic applications can also benefit from using the oligonucleotides, conjugates or constructs defined herein.

Thus, a seventh aspect provides a therapeutic product which is or comprises an oligonucleotide according to the first aspect or a composition thereof, or a conjugate according to the second aspect or a composition thereof, or a construct according to the third aspect or a composition thereof, for use in therapy, for example, for use in gene therapy, cell therapy, or vaccination. This aspect may be reworded as use of a therapeutic product which is or comprises an oligonucleotide according to the first aspect or a composition thereof, or a conjugate according to the second aspect or a composition thereof, or a construct according to the third aspect or a composition thereof, for the preparation of a medicament. The invention also refers to a method of treatment comprising administering an effective amount of a therapeutic product which is or comprises an oligonucleotide according to the first aspect or a composition thereof, or a conjugate according to the second aspect or a composition thereof, or a construct according to the third aspect or a composition thereof, to a subject in need thereof.

According to an eighth aspect, the invention relates to the use in diagnosis of an oligonucleotide according to the first aspect or a composition thereof, or a conjugate according to the second aspect or a composition thereof, or a construct according to the third aspect or a composition thereof.

According to a ninth aspect, the invention relates to the use in cosmetics of an oligonucleotide according to the first aspect or a composition thereof, or a conjugate according to the second aspect or a composition thereof, or a construct according to the third aspect or a composition thereof.

According to a tenth aspect, the invention relates to a kit for delivering a cargo component to a cell, in particular to the nucleus of a cell, the kit comprising an oligonucleotide according to the first aspect or a composition thereof, or a conjugate according to the second aspect or a composition thereof, or a construct according to the third aspect or a composition thereof.

### Brief Description of Drawings

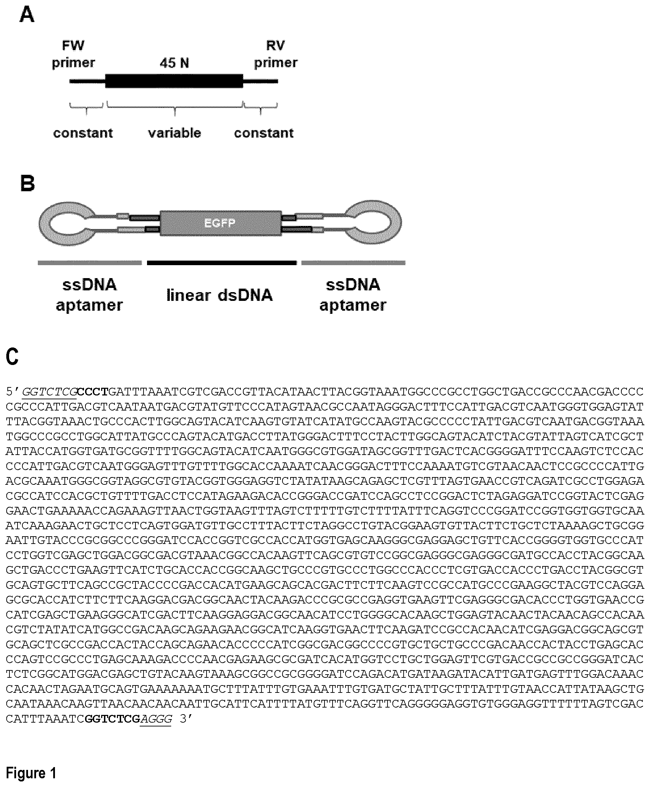

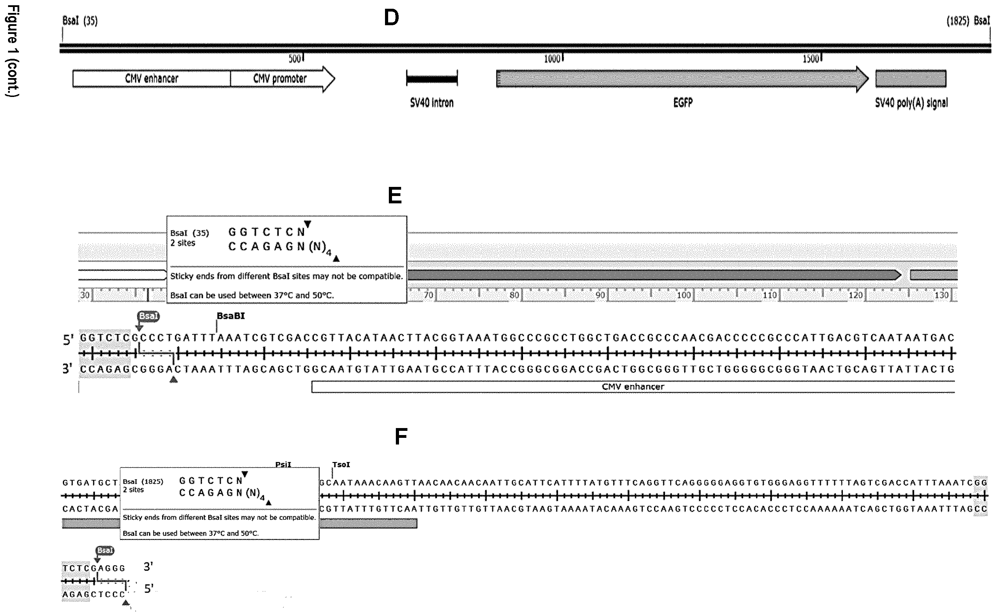

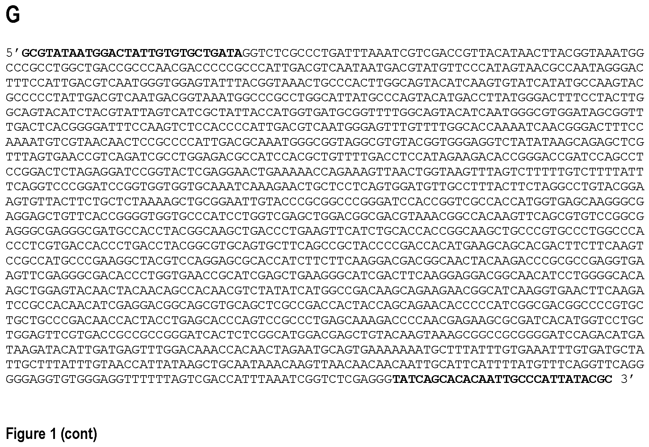

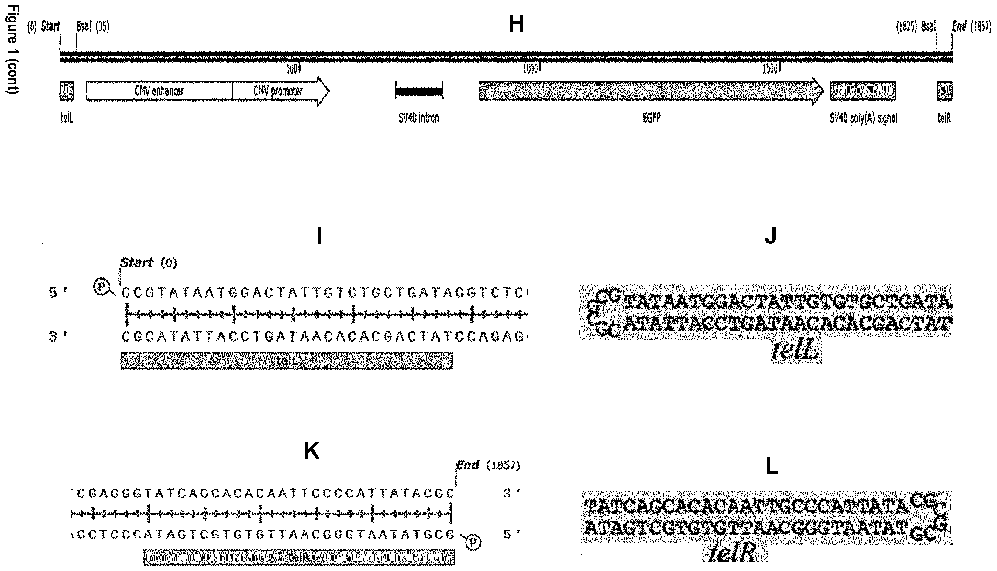

**Figure 1****.** DNA aptamer species. (A) Structure of ssDNA aptamers, including a variable region of 45 nucleotides and 2 constant regions for specific primer annealing which allow PCR amplification. (B) Structure of oDNA: linear double stranded DNA (dsDNA) encoding the Gene of Interest; in this case is the EGFP gene, closed at the ends with ssDNA hairpin aptamers. (C) DNA sequence with Gene of Interest coding for EGFP for preparing oDNAs (SEQ ID NO: 173). (D) Cartoon representation of the DNA sequence (SEQ ID NO: 173) with Gene of Interest coding for EGFP as well as recognition sites for Bsal. (E) 5' end of the sequence above where Bsal enzyme cuts keeping "CCTC" at 5' (as illustrated in 1C, bolded nucleotides are kept and nucleotides in italics as well as underlined are removed). (F) 3' end of the sequence above where Bsal enzyme cuts removing "AGGG" at 3' (as illustrated in 1C, bolded nucleotides are kept and nucleotides in italics as well as underlined are removed). (G) DNA sequence with Gene of Interest coding for EGFP and containing target sequences for protelomerase TelN (SEQ ID NO: 174), nucleotides in bold, for preparing control IcDNA. (H) Cartoon representation of the linear DNA sequence of SEQ ID NO: 174 with Gene of Interest coding for EGFP as well as TelN targeting sequences. (I) 5' end of the SEQ ID NO: 174. (J) Closed end formed at the 5' end after using TelN. (K) 3' end of the SEQ ID NO: 174. (L) Closed end formed at the 3' end after using TeIN.

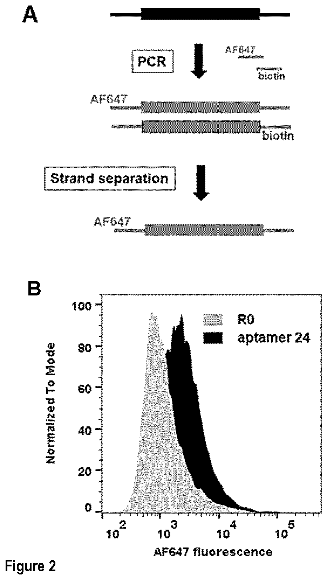

**Figure 2****.** Flow cytometry assays for aptamer internalization. (A) Labelling protocol of aptamers. The ssDNA aptamers were amplified by PCR with Alexa Fluor 647 (AF647)-labelled forward primer and biotinylated reverse primer. Strand separation was carried out using streptavidin Sepharose columns. (B) Representative histograms of cells incubated with reference control (R0) and aptamer 24.

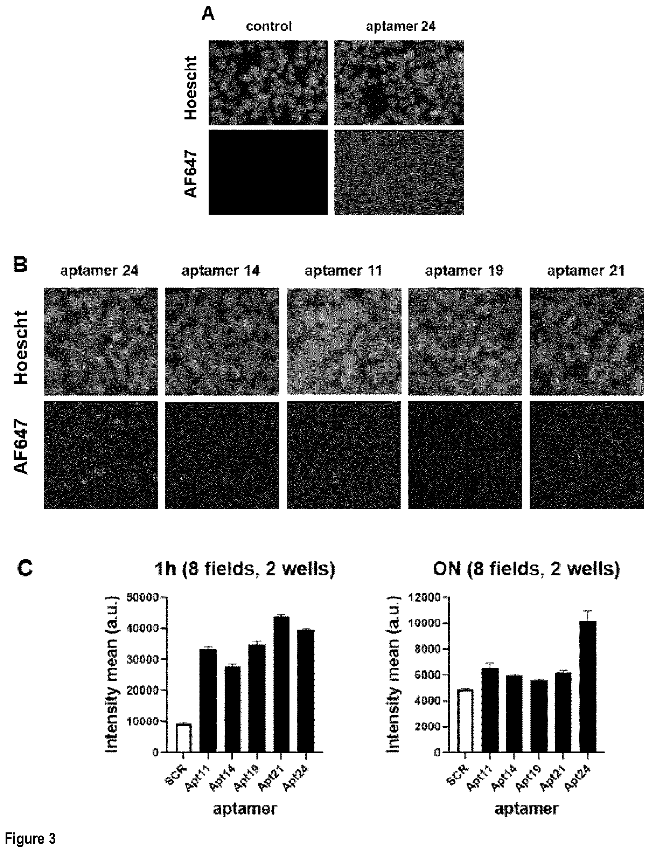

**Figure 3****.** Fluorescence microscopy assays for aptamer internalization. HEK293T cells were incubated with a selection of aptamers for 1 hour or overnight (ON). (A) Representative images of control cells and aptamer 24-treated cells after 1 hour. (B) Representative images of cells after overnight incubation with different aptamers. (C) Graphs showing the quantification of AF647 fluorescence in nuclei (Hoescht) for control (SCR) and aptamers. Bars represent the intensity mean for all nuclei in each field.

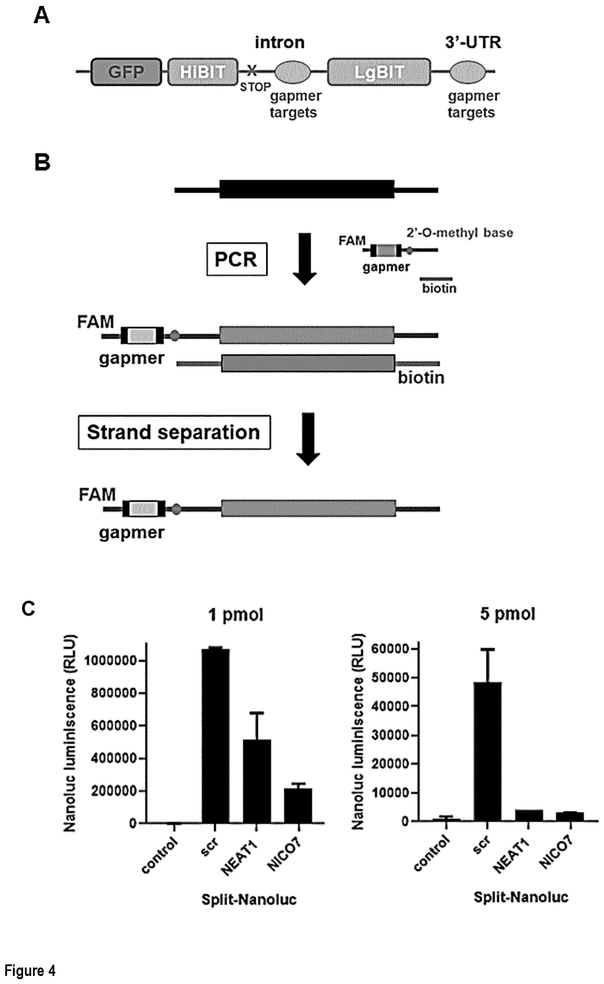

**Figure 4****.** Gapmer-aptamer internalization assays and Split-Nanoluc reporter system. (A) Split-Nanoluc construct used as reporter system. (B) Protocol for gapmer-aptamer synthesis. The ssDNA aptamers were amplified by PCR with gapmer-containing forward primer and biotinylated reverse primer. Strand separation was carried out using streptavidin sepharose columns. (C) HEK293T cells were transfected with Split-Nanoluc reporter plasmid and 1 or 5 pmol of gapmer-aptamer24 (scr: scrambled sequence). After 24 hours, cells were collected and Nanoluc-derived luminescence was measured with Nano-Glo Luciferase Assay.

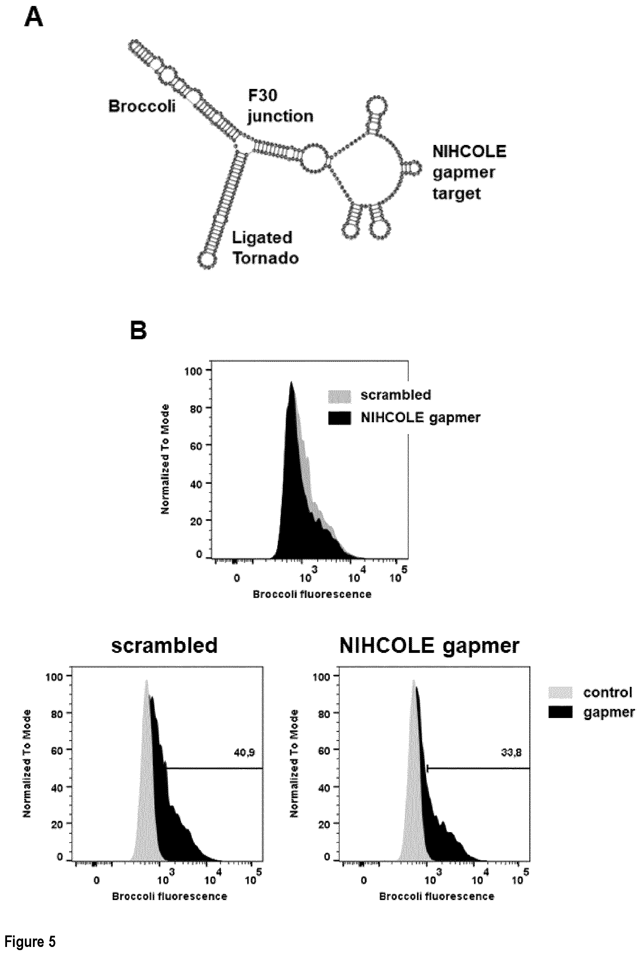

**Figure 5****.** Gapmer-aptamer internalization assays and Broccoli RNA reporter system. (A) Structure of the circular Broccoli RNA used as reporter system for gapmer internalization. (B) Stable HEK293T cells expressing this RNA molecule were incubated with 10 pmol of gapmers-aptamer24. After 24 hours, cells were collected, incubated in the presence of the DFHBI-1T compound and Broccoli fluorescence was measured in a flow cytometer. Representative histograms showing the difference between NIHCOLE gapmer and a scrambled sequence.

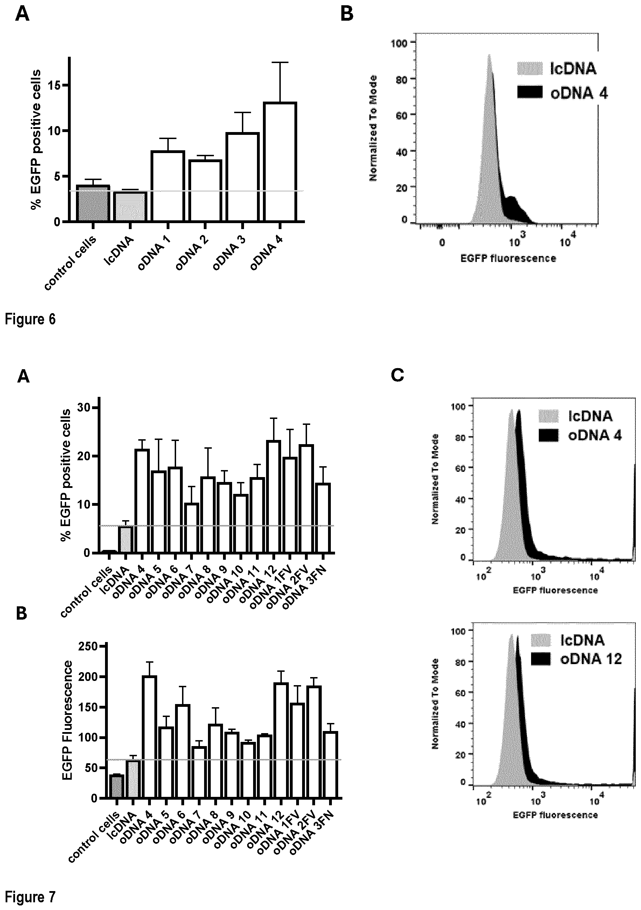

**Figure 6****. Free uptake experiments of oDNA.** A) HEK293 cells were incubated with medium alone (control cells), control IcDNA, oDNAs (1-4). After 24 hours, EGFP fluorescence in cells was analysed by flow cytometry. Graph show the percentage of EGFP positive cells for all samples. B) Representative histograms evaluating GFP levels of cells incubated with control (IcDNA) and oDNA4.
**Figure 7****. JetPEI-based transfection experiments of oDNA.** HEK293T cells were transfected with oDNAs or a linear DNA closed with a small oligonucleotide (linear closed DNA or IcDNA). After 12 hours, cells were collected and cellular fluorescence was measured in a flow cytometer or a fluorimeter. (A) Graph shows the percentage of EGFP positive cells measured in a flow cytometer. (B) Graph shows the mean of cellular EGFP fluorescence measured in a fluorimeter. (C) Representative histograms of cells incubated with control (IcDNA) and oDNA 4 or oDNA 12 of flow cytometry analysis. The *x* axis represents fluorescence intensity, and the y axis the normalize number of acquired cells per sample.

### Detailed description of the invention

### Definitions

All terms as used herein in this application, unless otherwise stated, shall be understood in their ordinary meaning as known in the art. Other more specific definitions for certain terms as used in the present application are as set forth below and are intended to apply throughout the description and claims.

The term "about" or "around" as used herein refers to a range of values ± 10% of a specified value. For example, the expression "about 10" or "around 10" includes ± 10% of 10, i.e. from 9 to 11.

As used herein, the indefinite articles "a" and "an" are synonymous with "at least one" or "one or more." Unless indicated otherwise, definite articles used herein, such as "the" also include the plural of the noun.

To facilitate understanding and clarify the meaning of specific terms in the context of the present invention, the following definitions and particular and preferred embodiments thereof, applicable to all the embodiments of the different aspects of the present invention, are provided:
The term "nucleotide" in the sense of the present invention refers to any type of nucleotide, including but not limited to deoxyribonucleotides (containing 2'-deoxy-D-ribose) and ribonucleotides (containing D ribose).

According to the present disclosure the term "nucleotide" includes not only those with conventional nucleobases, sugar residues and phosphate groups, but also those that contain modifications of any or all of these three moieties (hereinafter also referred as "modified nucleotides"). In the sense of the present invention the modified nucleotides may include "nucleotide analogues", which are compounds which are analogous (structurally similar) to naturally occurring deoxyribonucleotides or ribonucleotides, used in medicine and in molecular biology research. As used herein, the term "nucleotides" include conventional and non-conventional nucleotides, natural and non-natural nucleotides.

"Oligonucleotides" are generally recognised as short nucleotide sequences. The length of the oligonucleotide sequence is not clear-cut but is usually considered below 200, while often they have around 30-60 nucleotides. In the sense of the present disclosure, the term oligonucleotide is to be interpreted in its broad sense as a nucleotide sequence of up to 200 nucleotides. Further, in the sense of the present disclosure, said oligonucleotides may contain conventional nucleotides (i.e. deoxyribonucleotides and/or ribonucleotides), modified nucleotides, or nucleotide analogues. In the sense of the present invention, oligonucleotides may be oligodeoxyribonucleotides, oligoribonucleotides, oligonucleotides containing modified nucleotides, or mixtures thereof. The nucleotide sequences according to the invention may also contain non-conventional inter-nucleotide linkages.

Nucleic acids are polymeric macromolecules assembled from nucleotides. DNA (deoxyribonucleic acid) and RNA (ribonucleic acid) are large chains (sequences) of deoxyribonucleotides or ribonucleotides, respectively. In the sense of the present invention, nucleic acids may contain DNA sequences, RNA sequences, modified nucleotide sequences, analogues, or mixtures thereof.

Sequence identity, including determination of sequence complementarity for nucleic acid sequences and sequence similarity, may be determined by sequence comparison and alignment algorithms known in the field. To determine the percent identity of two nucleic acid sequences (or of two amino acid sequences), the sequences are aligned for optimal comparison purposes (e.g., gaps can be introduced in the first sequence or second sequence for optimal alignment). The nucleotides (or amino acid residues) at corresponding nucleotide (or amino acid) positions are then compared. When a position in the first sequence is occupied by the same residue as the corresponding position in the second sequence, then the molecules are identical at that position. The percent identity between the two sequences is a function of the number of identical positions shared by the sequences (i.e., % homology=# of identical positions/total # of positions* 100), optionally penalizing the score for the number of gaps introduced and/or length of gaps introduced. Similarity in sequence alignment is the resemblance between two sequences when compared. This fact is dependent on the identity of sequences. Similarity depicts the extent to which the residues are aligned. Hence, similar sequences contain similar properties.

The comparison of sequences and determination of percent identity or similarity between two sequences can be accomplished using a mathematical algorithm. In one embodiment, the alignment is a local alignment. A preferred, non- limiting example of a local alignment algorithm utilized for the comparison of sequences is the algorithm of Karlin and Altschul (1990) Proc. Natl. Acad. Sci. USA 87:2264-68, modified as in Karlin and Altschul (1993) Proc. Natl. Acad. Sci. USA 90:5873-77. Such an algorithm is incorporated into the BLAST programs (version 2.0) of Altschul, et al. (1990) J. Mol. Biol. 215:403-10. In another embodiment, the alignment is optimized by introducing appropriate gaps and percent identity or similarity is determined over the length of the aligned sequences (i.e., a gapped alignment). To obtain gapped alignments for comparison purposes, Gapped BLAST can be utilized as described in Altschul et al, (1997) Nucleic Acids Res. 25(17):3389-3402. In another embodiment, the alignment is optimized by introducing appropriate gaps and percent identity or similarity is determined over the entire length of the sequences aligned (i.e., a global alignment). A preferred, non-limiting example of a mathematical algorithm utilized for the global comparison of sequences is the algorithm of Myers and Miller, CABIOS (1989). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The term "coupled to" as used herein is intended to encompass any complex (conjugate or construct) wherein the oligonucleotide of the first aspect is linked, attached or joined, directly or indirectly, to a cargo component as described herein. Methods for effecting coupling will be known to the skilled in the art and include, but are not limited to conjugation, linking via peptide or nucleotide linker or by direct chemical synthesis of the oligonucleotide and cargo component as a whole chain.

As used herein, the term "treat" or "treatment" or "treating" shall be understood to mean administering an effective amount of any of the products disclosed herein and reducing or inhibiting at least one symptom of a clinical condition or disease. In the sense of the present invention, the term "treat" or "treatment" or "treating" also encompasses prophylactic treatment.

The terms "prophylactic treatment" shall be taken to mean administering an effective amount of any of the products disclosed herein and stopping, preventing, hindering or delaying, totally or partially, the development or progression of at least one symptom of a disease.

The expression "therapeutically effective amount" refers to sufficient quantity of any of the products according to the present invention to treat a particular condition. The skilled person will be aware that such an amount will vary depending upon, for example, the particular subject and/or the type or severity or level of disease. The term is not be construed to limit the present disclosure to a specific quantity of the products disclosed herein.

As used herein, the term "subject" shall be taken to mean any subject, including a human or non-human subject. The non-human subject may include non-human primates, ungulate (bovines, porches, ovines, caprines, equines, buffalo and bison), canine, feline, lagomorph (rabbits, hares and pikas), rodent (mouse, rat, guinea pig, hamster and gerbil), avian, and fish. Preferably, the subject is a human.

### Oligonucleotides

As mentioned above, in a first aspect the invention provides an oligonucleotide comprising a sequence selected from the group consisting of SEQ ID NO: 1 to SEQ ID NO: 156. These oligonucleotides have been found to effectively translocate to the cell nucleus. The first aspect also refers to oligonucleotides comprising a sequence having at least 80% identity with a sequence selected from the group consisting of SEQ ID NO: 1 to SEQ ID NO: 156 and capable of translocating to the cell nucleus.

In one embodiment, the oligonucleotide according to the first aspect comprises a sequence selected from the group consisting of SEQ ID NO: 1 to SEQ ID NO: 151 or comprises a sequence having at least 80% identity with a sequence selected from the group consisting of SEQ ID NO: 1 to SEQ ID NO: 151 and capable of translocating to the cell nucleus. In another embodiment, the oligonucleotide comprises a sequence selected from the group consisting of SEQ ID NO: 152, SEQ ID NO: 153, SEQ ID NO: 154, SEQ ID NO: 155, and SEQ ID NO: 156.

In one embodiment, the oligonucleotide of the first aspect comprises a sequence selected from the group consisting of SEQ ID NO: 1 to SEQ ID NO: 50. In a particular embodiment, the oligonucleotide comprises a sequence selected from the group consisting of SEQ ID NO: 1 to SEQ ID NO: 25. In a more particular embodiment, the oligonucleotide comprises a sequence selected from the group consisting of SEQ ID NO: 6, SEQ ID NO: 7, SEQ ID NO: 10, SEQ ID NO: 11, SEQ ID NO: 14, SEQ ID NO: 19, SEQ ID NO: 21, SEQ ID NO: 23, and SEQ ID NO: 24.In a very particular embodiment the oligonucleotide comprises SEQ ID NO: 24.

In another particular embodiment, the oligonucleotide comprises sequence selected from the group consisting of SEQ ID NO: 1 to SEQ ID NO: 12. In a very particular embodiment, the oligonucleotide comprises a sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 8 and SEQ ID NO: 12. In another particular embodiment, the oligonucleotide comprises a sequence selected from the group consisting of SEQ ID NO: 152, SEQ ID NO: 153, SEQ ID NO: 154, SEQ ID NO: 155, and SEQ ID NO: 156. In another very particular embodiment, the oligonucleotide comprises a sequence selected from the group consisting of SEQ ID NO: 153, SEQ ID NO: 154, and SEQ ID NO: 155. In some embodiments, the oligonucleotide of the first aspect comprises a sequence having at least 80% identity with a sequence selected from any one of the sequences mentioned herein, i.e. a sequence selected from the group consisting of SEQ ID NO: 1 to SEQ ID NO: 12; or to a sequence selected from the group consisting of SEQ ID NO: 6, SEQ ID NO: 7, SEQ ID NO: 10, SEQ ID NO: 11, SEQ ID NO: 14, SEQ ID NO: 19, SEQ ID NO: 21, SEQ ID NO: 23, and SEQ ID NO: 24; or to a sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 8 and SEQ ID NO: 12; or to a sequence selected from the group consisting of SEQ ID NO: 152, SEQ ID NO: 153, SEQ ID NO: 154, SEQ ID NO: 155, and SEQ ID NO: 156; or to a sequence selected from the group consisting of SEQ ID NO: 153, SEQ ID NO: 154, and SEQ ID NO: 155.

In particular embodiments the sequence identity is at least 85%, or at least 90% or at least 95%, or at least 96%, or at least 97%. In more particular embodiments the sequence identity is at least 98% or 99%. Preferably, all the oligonucleotides having the mentioned identities have the ability to translocate to the cell nucleus.

In some embodiments, the oligonucleotides of the first aspect further comprise the sequences
ATCCAGAGTGACGCAGCA (SEQ ID NO: 157) and TGGACACGGTGGCTTAGT (SEQ ID NO: 158) flanking a sequence selected from SEQ ID NO: 1 to SEQ ID NO: 151. In particular embodiments, SEQ ID NO: 157 is located upstream from the sequence selected from SEQ ID NO: 1 to SEQ ID NO: 151, and SEQ ID NO: 158 is located downstream from the sequence selected from SEQ ID NO: 1 to SEQ ID NO: 151. In particular embodiments, SEQ ID NO: 157 is located directly upstream from the sequence selected from SEQ ID NO: 1 to SEQ ID NO: 151, and SEQ ID NO: 158 is located directly downstream from the sequence selected from SEQ ID NO: 1 to SEQ ID NO: 151. "Located upstream" a particular sequence is understood as located at the 5' end of said sequence. "Located downstream" a particular sequence is understood as located at the 3' end of said sequence. "Directly upstream" or "directly downstream" is understood as being located immediately contiguous to the 5' or 3' end of a particular sequence, respectively.

In another particular embodiment, the oligonucleotide of the first aspect comprises a sequence selected from the group consisting of SEQ ID NO: 159 to SEQ ID NO: 170, or a sequence having at least 80% identity with a sequence selected from the group consisting of SEQ ID NO: 159 to SEQ ID NO: 170 and capable of translocating to the cell nucleus.

The embodiments directed to oligonucleotides comprising any of the sequences mentioned above encompass, and can also be worded with, "consisting of" any one of the sequences.

In particular embodiments, the oligonucleotides of the first aspect are single stranded. In more particular embodiments, the oligonucleotides of the first aspect are oligodeoxyribonucleotides. In preferred embodiments, the oligonucleotides of the first aspect are single stranded oligodeoxyribonucleotides. In particular embodiments, the oligonucleotides of the first aspect are aptamers.

In particular embodiments, the oligonucleotides of the first aspect have a length from 35 to 100 nucleotides. In other particular embodiments, the oligonucleotides of the first aspect have a length from 35 to 80 nucleotides, for example from 35 to 60 nucleotides.

In some embodiments, the oligonucleotides of the first aspect comprise modified nucleotides. Such modified oligonucleotides are sometimes preferred over conventional nucleotides because of properties such as, for example, enhanced cellular uptake, reduced immunogenicity, and increased stability. The following paragraphs describe non-limiting modifications to the oligonucleotides.

In some embodiments, the oligonucleotides contain a phosphorothioated backbone. For example, none, 5 or more, 10 or more, 15 or more, 20 or more, 25 or more, 30 more more, 35 or more, 40 or more, 45 or more, or all of the oligonucleotides have a phosphorothioated backbone. Oligonucleotides that contain phosphorothioated backbones provide an increased resistance to nucleases compared to unmodified oligonucleotides. In some embodiments, the oligonucleotides comprise modifications to help enhance their properties. Hence, in some embodiments the oligonucleotide may be modified by the substitution of at least one nucleotide with at least one modified nucleotide, ideally so that the in vivo and in vitro stability of the oligonucleotide is enhanced as compared to a corresponding unmodified oligonucleotide. In some embodiments, the oligonucleotides comprise 2'-deoxy guanosine, 2'-deoxy adenosine, 2'-0-methylguanosine, 2'-0-methyl (e.g., 2'- O-methylcytidine, 2'-0-methylpseudouridine, 2'-0-methyluridine, 2'-0-methyladenosine, 2'-0-methyl) ribonucleotide, 2'-amino, 2'-thio and 2'-fluoro modified ribonucleotide, 2'-fluoro-cytidine, 2'-fluoro-uridine, 2'-fluoro-guanosine, 2'-fluoro-adenosine, 2'-amino-cytidine, 2'-amino-uridine, 2'-amino- adenosine, 2'-amino-guanosine, 2'-amino-butyryl-pyrene-uridine, 2'-amino-adenosine, 5-iodo-uridine, ribo-thymidine, 5-bromo-uridine, 2-aminopurine, 5-methyl-cytidine , 5-fluoro-cytidine, and 5-fluoro-uridine, 2,6-diaminopurine, 4-thio-uridine, and/or 5-amino-allyl-uridine.

In some embodiments, the oligonucleotides include derivatization of the 5 position, for instance being selected from 5-(2-amino) propyl uridine, 5-bromo uridine, 5-propyne uridine, 5-propenyl uridine; derivatization of the 6 position, for instance 6-(2-amino)propyl uridine; derivatization of the 8-position for adenosine and/or guanosines, for instance 8- bromo guanosine, 8-chloro guanosine, or 8-fluoroguanosine. In other embodiments, the oligonucleotides comprise nucleotide analogs such as deaza nucleotides, e.g., 7-deaza-adenosine; O- and N-modified (for instance alkylated, such as N6-methyl adenosine) nucleotides; and other heterocyclically modified nucleotide analogs.

In other embodiments, the oligonucleotides comprise a modified sugar residue. Examples of modifications to the sugar residue of the nucleotides which may be employed include the 2' OH-group being replaced by a group selected from H, OR, R, F, Cl, Br, I, SH, SR, H2, NHR, NR2, COOR, or OR, wherein R is substituted or unsubstituted C₁-C₆ alkyl, alkenyl, alkynyl, aryl and so on. The phosphate group of the nucleotide may also be modified, such as by substituting one or more of the oxygens of the phosphate group with sulphur (for instance by employing phosphorothioates). Modifications may decrease the rate of hydrolysis of oligonucleotides, for example by inhibiting degradation by exonucleases. In one preferred instance, the oligonucleotide is resistant to ribonucleases. Oligonucleotides which may be employed include those with modifications to promote such resistance, for instance an oligonucleotide of the invention may have preferably been modified with a 2'- O-methyl group (e.g., 2'-0-methylcytidine, 2'-0-methylpseudouridine, 2'-0-methylguanosine, 2'-0-methyluridine, 2'-0-methyladenosine, 2'-0-methyl). In certain embodiments, the oligonucleotides contain a modification to increase resistance to ribonucleases and a phosphorothioate backbone.

In other embodiments, the oligonucleotides contain 5-fluorouracil, 5-bromouracil, 5-chlorouracil, 5-iodouracil, hypoxanthine, xanthine, 4-acetylcytosine, 5-(carboxyhydroxylmethyl)uracil, 5- carboxymethylaminomethyl-2-thiouridine, 5-carboxymethylaminomethyluracil, dihydrouracil, beta-D-galactosylqueosine, inosine, N6-isopentenyladenine, 1- methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D- mannosylqueosine, 5'-methoxycarboxymethyluraci 1, 5-methoxyuracil, 2-methylthio-N6- isopentenyladenine, uracil-5-oxyacetic acid (v), wybutoxosine, pseudouracil, queosine, 2- thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino- 3-N-2-carboxypropyl) uracil, (acp3)w, and 2,6-diaminopurine.

In some advantageous embodiments the oligonucleotide contains peptide nucleic acid (PNA), Morpholino nucleic acid, glycol nucleic acid (GNA), threose nucleic acid (TNA), hexitol nucleic acids (HNA).

In some embodiments, the oligonucleotides include modifications to the phosphate backbone such as methyl phosphonates, methyl phosphonothioates, phosphoromorpholidates, phosphoropiperazidates and phosphoramidates.

In other embodiments, the modified nucleotide may be an abasic site. As used herein, an "abasic site" is a nucleotide lacking the organic base. In preferred embodiments, the abasic nucleotide further comprises a chemical modification as described herein at the 2' position of the ribose.

The oligonucleotides of the invention may be prepared by methods well known in the art. For example, the oligonucleotides may be prepared by chemical synthesis from activated nucleotides.

### Conjugates and constructs

The oligonucleotides of the first aspect can be conjugated to other molecules or group of molecules (a cargo) and deliver said molecule or group of molecules inside a cell, in particular, they can deliver said molecule or group of molecules to the cell nucleus. Therefore, a second aspect provides a conjugate comprising at least one delivery component and at least one cargo component, wherein the at least one delivery component comprises an oligonucleotide as defined in the first aspect.

The conjugates of the second aspect may contain more than one oligonucleotide as defined in the first aspect. For example, the conjugates may contain two, three, four or more oligonucleotides according to the first aspect.

The cargo component may be any molecule or group of molecules, such as a drug (small molecule or biologic), a protein or a gene, or it can be a metal, a radioisotope, or any other compound which needs to be delivered to the cell, in particular to the cell nucleus. All embodiments defined above for the oligonucleotides of the first aspect also apply to the conjugates of the second aspect.

The cargo component may be located upstream or downstream the oligonucleotide of the first aspect and may be coupled directly or via a linker to the 5' or 3'-end of the oligonucleotide of the first aspect. In a particular embodiment, the cargo component is coupled to the 3'-end of the oligonucleotide of the first aspect. In another particular embodiment, the cargo component is located between two oligonucleotides of the first aspect. The coupling between the delivery component and the cargo component in the conjugate can be a covalent bond or a non-covalent bond. In some embodiments, the delivery component and the cargo component are separated by a spacer (or linker). The nature of the spacer or linker is not particularly limited, and can be, for example, a peptide, a chemical bond, or a nucleotide sequence. The conjugates described herein may be prepared by any method known in the art.

In one embodiment of the second aspect, the cargo component comprises a molecule selected from the group consisting of peptides, proteins, antibodies, amino acids, enzymes, lipids, fatty acids, carbohydrates, sugars, and small molecules. In some embodiments the cargo component may comprise an inorganic compound, such as a metal. In particular embodiments, the cargo component comprises an antibody. In another particular embodiment the cargo component comprises an enzyme, such as horseradish peroxidase or alkaline phosphatase. In other particular embodiments, the cargo component comprises a small molecule, such as, but not limited to, a chemotherapeutic agent, for example, doxorubicin, gemcitabine, docetaxel, trabectedin, temozolomide, eribuline and combinations thereof. In other particular embodiments, the cargo component may comprise an affinity molecule, such as biotin or streptavidin. In other particular embodiments the cargo component may comprise a molecule or substance that emits a detectable signal (also called a "label" or a "marker"). Examples of labels are fluorescent substances, luminescent substances, radioisotopes, enzymes, prosthetic groups, electron dense labels, labels for magnetic resonance imaging, and radioactive substances. The cargo component may contain one or more of the above molecules or substances.

The cargo component may also be a nucleic acid molecule (oligonucleotide or polynucleotide). When the cargo component is a nucleic acid molecule the conjugate is herein termed "construct". The invention thus provides, in a third aspect, a nucleic acid construct comprising at least one delivery component and at least one cargo component, wherein the delivery component comprises an oligonucleotide as defined in the first aspect and the cargo component is a polynucleotide or oligonucleotide. All embodiments defined above for the oligonucleotides of the first aspect also apply to the constructs of the third aspect.

The poly or oligonucleotides may contain deoxyribonucleotides, deoxyribonucleotide analogues, modified deoxyribonucleotides, or a combination thereof. The delivery component and the cargo component in the constructs of the invention may be separated by a spacer which is preferably a nucleotide sequence. The constructs described herein may be prepared by methods well known in the art.

In one embodiment of the second or third aspects, the cargo component does not comprise a deoxyribonucleotide sequence of more than 35 nucleotides. In one embodiment, the cargo component in the construct of the third aspect comprises or consists of a nucleotide sequence selected from a ribonucleotide sequence, with or without modified ribonucleotides, a deoxyribonucleotide sequence of a size equal or below 35 nucleotides, with or without modified deoxyribonucleotides, and combinations of the foregoing. In an equivalent embodiment, the cargo component comprises or consists of: a) a ribonucleotide sequence; b) a deoxyribonucleotide sequence, provided said deoxyribonucleotide sequence comprises 35 deoxyribonucleotides or less; or c) a combination of a) and b). In these embodiments, the cargo component preferably comprises or consists of: (i) a microRNA (mRNA), an interference oligonucleotide, a small interfering RNA (siRNA), an antisense oligonucleotide (AON), an aptamer, a guide RNA, a shRNA, an RNA or DNA structural domain, an RNA coding sequence, or a ribozyme; or (ii) a moiety selected from a radionuclide, a chemotherapeutic agent and combinations thereof, preferably a chemotherapeutic agent. Preferably, the cargo component comprises a siRNA, AON, microRNA, or a combination thereof.

Since RNAi and AON technology is readily adaptable to inhibit the expression of virtually any gene in the human genome, it has become a valuable tool for elucidating pathogenic mechanisms, such as deregulated cell growth and survival during malignancy. Furthermore, its potential use as therapeutic tool has also become apparent and highly pursued. However, despite recent developments, to date there are few approved AON or siRNA-based therapies, being delivery one of the major problems for the successful translation of these technologies into effective therapies for use in the clinical practice. Similarly, CIRSPR-Cas gene editing technology is opening the door for an increasing number of gene therapy applications but again requires effective nuclear translocation. The present invention provides efficient delivery of cargo molecules, such as siRNAs, AONs, guide RNAs, or chemotherapeutic drugs to the cell and, very particularly, to the cell nucleus, thus improving the efficiency of the therapeutic approach.

In one embodiment, the cargo component in the construct of the third aspect comprises or consists of a deoxyribonucleotide sequence of more than 35 nucleotides. In a particular embodiment, the cargo component comprises a deoxyribonucleotide sequence of more than 35 nucleotides comprising a sequence of interest. In this embodiment the sequence of interest may preferably be a double stranded DNA fragment that comprises the minimum necessary sequences encoding for a gene of interest. In preferred embodiments, the sequence of interest further comprises additional sequences that are required for correct gene expression. For example, the sequence of interest may be an expression cassette. The term "expression cassette" refers to a DNA sequence comprising one or more promoter or enhancer elements and a gene or other coding sequences, preferably encoding a protein of interest. The expression cassette may further comprise other elements that regulate the expression of the coding sequence, such as a transcription termination site.

In some embodiments, the cargo component may additionally comprise other sequences flanking the expression cassette, such as inverted terminal repeats (ITRs). In a particular embodiment, the inverted terminal repeats (ITR) are flanking the gene of interest. In a particular embodiment the ITRs are comprised flanking an expression cassette. As used herein, the term "terminal repeat" or "TR" includes any viral terminal repeat or synthetic sequence that comprises at least one minimal required origin of replication and a region comprising a palindrome hairpin structure. A Rep-binding sequence ("RBS") (also referred to as RBE (Rep-binding element)) and a terminal resolution site ("TRS") together constitute a "minimal required origin of replication" and thus the TR comprises at least one RBS and at least one TRS. TRs that are the inverse complement of one another within a given stretch of polynucleotide sequence are typically each referred to as an "inverted terminal repeat" or "ITR".

In a particular embodiment, the construct according to the third aspect is a closed linear DNA (clDNA). The cIDNA according to this embodiment consists of a stem region comprising a double stranded gene of interest, wherein the stem region is covalently closed at both ends by hairpin loops, wherein the hairpin loops comprise an oligonucleotide as defined in the first aspect. In some embodiments, each loop comprises an oligonucleotide according to the first aspect.

As used herein, the term "closed linear DNA" or "clDNA" (also termed in the examples herein as oDNA) refers to a single stranded covalently closed DNA molecule that forms a "dumbbell" or "doggy-bone" shaped structure under conditions allowing nucleotide hybridization. Therefore, although the cIDNA is formed by a single stranded DNA molecule, the formation of the "dumbbell" structure by the hybridization of two complementary sequences within the same molecule generates a structure consisting on a double-stranded middle segment flanked by two single-stranded loops. The skilled in the art knows how to generate cIDNA from open or closed double stranded DNA using routine molecular biology techniques. For instance, the skilled in the art knows that a cIDNA can be generated by attaching hairpin DNA adaptors -for instance, by the action of a ligase- to both ends of an open double stranded DNA. "Hairpin DNA adaptor" refers to a single stranded DNA that forms a stem-loop structure by the hybridization of two complementary sequences, wherein the stem region formed is closed at one end by a single stranded loop and is open at the other end. In particular embodiments, the stem region comprises a deoxyribonucleotide sequence which is longer than 35 nucleotides comprising a sequence of interest, and at least one of the loops comprise or consist of an oligonucleotide according to the first aspect. In particular embodiments both loops comprise or consist of an oligonucleotide according to the first aspect.

The stem region of the cIDNA may comprise additional sequences that play any structural or functional role complementary to the gene of interest, or any other role that the cIDNA may require to fulfil its final object, for example, sequences selected from the group consisting of: i) two restriction sites flanking the gene of interest, ii) a primase recognition site, iii) one or more ITRs, and iv) combinations thereof. In particular embodiments, the stem region comprises an expression cassette. Said expression cassette comprises a gene of interest integrated in the expression cassette. In some embodiments, the cIDNA comprises the following elements operably linked and in the following order: at least one promoter, optionally at least one 5' untranslated region sequence, at least one gene of interest, optionally at least one 3' untranslated region sequence, and at least one transcriptional terminator sequence, with suitable DNA spacer sequences optionally placed between the aforementioned elements.

In a particular embodiment, the cIDNA further comprises a primase/polymerase priming site. The primase recognition site may be present, for example, in the stem region. In a particular embodiment the primase recognition site is comprised in at least one of the loops. In another particular embodiment of the first aspect of the invention, optionally in combination with any of the embodiments provided above or below, the cIDNA does not comprise a primase/polymerase priming site. By including a primase recognition site, it is facilitated the use of a primase for priming the amplification of the cIDNA of the invention. The cIDNA products defined herein may be prepared by methods known to the skilled person. For example, methods to prepare cIDNA are described in WO2021/152146 and WO 2021/152147.

In some embodiments, the cargo component of the constructs of the invention comprises modified oligonucleotides.

In some embodiments, the conjugate or construct of the invention is joined directly, or indirectly via a linker, to further molecule. In particular embodiments, said further molecule is a label (for example a fluorogenic or colorimetric molecule), a metal nanoparticle, a polymer, a molecule bearing radioisotopes, a metal such as boron, or a liposome. The conjugate or construct and further molecule can also be bound via non-covalent bonds, such as by electrostatic or van-der-waals forces. The conjugate or construct and further molecule can form a complex. In particular embodiments, the further molecule is a polymer, and the complex is called a "polyplex".

### Compositions

The conjugates or constructs of the present invention can be used as, for example, in the form of a pharmaceutical composition. Thus, in a fourth aspect, the present invention refers to a composition comprising the oligonucleotides, conjugates, or constructs defined herein, together with one or more appropriate excipients or carriers. All embodiments defined above for the oligonucleotides of the first aspect, conjugates of the second aspect, or construct of the third aspect, also apply to the compositions of the fourth aspect.

In particular embodiments of the fourth aspect, the composition is a pharmaceutical composition and comprises a therapeutically effective amount of an oligonucleotide, conjugate, or construct defined herein, together with pharmaceutically acceptable excipients and/or carriers. The expression "excipients and/or carriers" refers to acceptable materials, compositions or vehicles. Each component must be pharmaceutically acceptable in the sense of being compatible with the other ingredients of the composition. It must also be suitable for use in contact with the tissue or organ of humans and non-human animals without excessive toxicity, irritation, allergic response, immunogenicity or other problems or complications commensurate with a reasonable benefit/risk ratio. Examples of suitable acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical or cosmetical composition, its use is contemplated to be within the scope of this invention.

The formulations of the pharmaceutical compositions described herein may be prepared by any method known or hereafter developed in the art of pharmacology. In general, such preparatory methods include the step of bringing the active ingredient (oligonucleotide, conjugate, or construct of the invention) into association with an excipient and/or one or more other accessory ingredients, and then, if necessary and/or desirable, shaping and/or packaging the product into a desired single- or multi-dose unit.

A pharmaceutical composition of the invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient.

The relative amounts of the active ingredient (oligonucleotide, conjugate or construct of the invention), the acceptable excipients, and/or any additional ingredients in the composition of the invention will vary, depending upon the identity, size, and/or condition of the subject treated and further depending upon the route by which the composition is to be administered.

Examples of the pharmaceutically acceptable carrier include, but are not limited to, excipients such as sucrose, starch, mannit, sorbit, lactose, glucose, cellulose, talc, calcium phosphate, and calcium carbonate; binders such as cellulose, methylcellulose, hydroxylpropylcellulose, polypropylpyrrolidone, gelatin, gum arabic, polyethylene glycol, sucrose, and starch; disintegrants such as starch, carboxymethylcellulose, hydroxylpropylstarch, sodium-glycol-starch, sodium hydrogen carbonate, calcium phosphate, and calcium citrate; lubricants such as magnesium stearate, Aerosil^{®}, talc, and sodium lauryl sulfate; flavoring agents such as citric acid, menthol, glycyrrhizin-ammonium salt, glycine, and orange powder; preservatives such as sodium benzoate, sodium hydrogen sulfite, methylparaben, and propylparaben; stabilizers such as citric acid, sodium citrate, and acetic acid; suspending agent such as methylcellulose, polyvinylpyrrolidone, and aluminum stearate; dispersant such as surfactants; diluents such as water, saline, and orange juice; base waxes such as cacao butter, polyethylene glycol, and white kerosene; and the like.

The composition of the present invention can be formulated in any form known by the skilled in the art suitable for the desired administration (e.g., oral, parenteral, inhalant). In particular embodiments the composition of the invention is formulated for parenteral administration, for example for intravenous or subcutaneous administration.

In other embodiments of the fourth aspect, the composition is a diagnostic composition.

### Uses of the products of the invention

According to the fifth aspect, it is provided a method for delivering a cargo component to a cell, said method comprising contacting a conjugate or construct as defined in the second or third aspects with the cell under conditions favourable for the uptake of said conjugate or construct by the cell. A sixth aspect relates to the use of an oligonucleotide as defined herein for cell-delivery of a cargo component. All embodiments defined above for the oligonucleotides of the first aspect, conjugates of the second aspect, constructs of the third aspect, also apply to the method of the fifth aspect and to the use of the sixth aspect.

In particular embodiments, the delivery is to the cell nucleus, i.e. the invention provides for method for nuclear-targeted delivery of a cargo component. The delivery or nuclear translocation to the cell can be *in vitro, ex vivo,* or *in vivo.* In more particular embodiments, the delivery or nuclear translocation takes place in an *in vivo* environment. In some embodiments, the favourable conditions for the uptake of said conjugate or construct by the cell comprise electroporation or integrating the nucleic acid construct into a transfection vector. In a very particular embodiment, the favourable conditions for the uptake of said conjugate or construct by the cell comprise physiological conditions, i.e. electroporation or integrating the construct into a transfection vector is not required for uptake of the conjugate or construct by the cell. In particular embodiments the favourable conditions comprise direct contact of the oligonucleotides, conjugates or constructs described herein with the cell.

As mentioned above, the oligonucleotides, conjugates and constructs of the invention may be used in medicinal, cosmetic and diagnostic applications.

In a seventh aspect, the invention provides a therapeutic product which consists of or comprises an oligonucleotide according to the first aspect or a composition thereof, or a conjugate according to the second aspect or a composition thereof, or a construct according to the third aspect or a composition thereof, for use in therapy. All embodiments defined above for the oligonucleotides of the first aspect, conjugates of the second aspect, constructs of the third aspect, and compositions of the fourth aspect, also apply to the seventh aspect.

In one embodiment, the therapeutic use may be related to the *in vitro, ex vivo* or, preferably, the *in vivo* expression in a host cell. In particular embodiments of the seventh aspect the products of the invention are for use in gene therapy, cell therapy, or vaccination.

In one embodiment the therapeutic use is vaccination by delivering to a cell, in particular to the cell nucleus, a cargo component encoding a modified form of an infectious organism's DNA/RNA. This embodiment can be reworded as a therapeutic use which comprises delivering a DNA/RNA to the cell, in particular, to the cell nucleus. DNA/RNA vaccines are administered to a subject where they then express the selected protein of the infectious organism, initiating an immune response against that protein which is typically protective. DNA/RNA vaccines may also encode a tumor antigen in a cancer immunotherapy approach.

Other therapeutic uses according to the seventh aspect are those related to gene therapy. For example, the products of the invention can be used to express a functional gene where a subject has a genetic disorder caused by a dysfunctional version of that gene. Alternatively, the products may be used to repair a dysfunctional gene underlying a genetic disorder. Non-limiting examples of such diseases include Duchenne muscular dystrophy, cystic fibrosis, Gaucher's Disease, and adenosine deaminase (ADA) deficiency. Other non-limiting examples of diseases where gene therapy may be useful include inflammatory diseases, autoimmune, chronic and infectious diseases, including such disorders as AIDS, cancer, neurological diseases, cardivascular disease, hypercholestemia, various blood disorders including various anaemias, thalassemia and haemophilia, and emphysema. For the treatment of solid tumors, genes encoding toxic peptides (i.e., chemotherapeutic agents such as ricin, diptheria toxin and cobra venom factor), tumor suppressor genes such as p53, genes coding for mRNA sequences which are antisense to transforming oncogenes, antineoplastic peptides such as tumor necrosis factor (TNF) and other cytokines, or transdominant negative mutants of transforming oncogenes, may be expressed.

The therapeutic use according to the seventh aspect may also be related to the expression of therapeutic proteins or peptides, such as therapeutic antibodies.

In other embodiments, the therapeutic use according to the seventh aspect is related to RNA interference therapy. Said therapeutic approach is mediated by an active RNA form, for example a siRNA, an AON, a small activating RNA (saRNA), a miRNA (or miRNA mimic), or an antimiR, all of which may be delivered to the cell, in particular, the nucleus of the cell, within the cargo component in the constructs of the invention. AON therapies involve short strands of (often modified) nucleotides that target RNA in a sequence - specific manner, inducing targeted protein knockdown or restoration. RNAi is a robust technique frequently employed to selectively suppress gene expression in a sequence-specific manner. A miRNA mimic behaves similarly to an endogenous miRNA, restoring or enhancing the function of a miRNA that may be lost or downregulated in a disease. Other gene regulating therapeutic approaches known to the skilled person may be improved by nuclear-targeted delivery provided by the present invention.

In other embodiments, the therapeutic use according to the seventh aspect is related to a small molecule or biologic drug which is delivered to a cell within the cargo component in the conjugates of the invention and targets a particular receptor or mechanism inside the cell, in particular, in the cell nucleus. The mechanism of action and nature of the drug is not particularly limited. Illustrative examples are chemotherapeutic drugs.

The present disclosure also provides the oligonucleotide, conjugate, construct, product, or composition as defined herein, together with a further therapeutic active agent for use in combination therapy, wherein the oligonucleotide, conjugate, construct, product, or composition as defined herein and the further therapeutic active agent may be administered concomitantly, sequentially, or separately within a therapeutic interval.

The eighth and ninth aspects of the invention refer to the use of the oligonucleotide, conjugate, construct, product or composition as defined herein in diagnosis and cosmetics, respectively. Moreover, the invention also refers to the use of the oligonucleotides, conjugates, constructs, products or compositions as defined herein as tools in molecular biology. All embodiments defined above for the oligonucleotides of the first aspect, conjugates of the second aspect, constructs of the third aspect, and compositions of the fourth aspect, also apply to these aspects.

For completeness, the present description is also disclosed in the following numbered embodiments:
1. An oligonucleotide comprising or consisting of:
   a sequence selected from the group consisting of SEQ ID NO: 1 to SEQ ID NO: 156, or
   a sequence having at least 80% identity with a sequence selected from the group consisting of SEQ ID NO: 1 to SEQ ID NO: 156 and is capable of translocating to the cell nucleus.
2. The oligonucleotide according to embodiment 1, comprising a sequence having at least 90% identity with a sequence selected from the group consisting of SEQ ID NO: 1 to SEQ ID NO: 156 and is capable of translocating to the cell nucleus.
3. The oligonucleotide according to embodiment 1, comprising a sequence having at least 95% identity with a sequence selected from the group consisting of SEQ ID NO: 1 to SEQ ID NO: 151 and is capable of translocating to the cell nucleus.
4. The oligonucleotide according to embodiment 1, comprising a sequence having at least 98% identity with a sequence selected from the group consisting of SEQ ID NO: 1 to SEQ ID NO: 151 and is capable of translocating to the cell nucleus.
5. The oligonucleotide according to embodiment 1, comprising a sequence having at least 99% identity with a sequence selected from the group consisting of SEQ ID NO: 1 to SEQ ID NO: 151 and is capable of translocating to the cell nucleus.
6. The oligonucleotide according to anyone of embodiments 1-5, wherein the sequence is selected from SEQ ID NO: 1 to SEQ ID NO: 50.
7. The oligonucleotide according to anyone of embodiments 1-5, wherein the sequence is selected from SEQ ID NO: 1 to SEQ ID NO: 25.
8. The oligonucleotide according to anyone of embodiments 1-5, wherein the sequence is selected from a group consisting of SEQ ID NO: 6, SEQ ID NO: 7, SEQ ID NO: 10, SEQ ID NO: 11, SEQ ID NO: 14, SEQ ID NO: 19, SEQ ID NO: 21, SEQ ID NO: 23, and SEQ ID NO: 24.
9. The oligonucleotide according to anyone of embodiments 1-5, wherein the sequence is selected from a group consisting of SEQ ID NO: 1 to SEQ ID NO: 12.
10. The oligonucleotide according to anyone of embodiments 1-5, wherein the sequence is selected from a group consisting of SEQ ID NO: 1, SEQ ID NO: 2, SEQ ID NO: 3, SEQ ID NO: 4, SEQ ID NO: 5, SEQ ID NO: 6, SEQ ID NO: 8, SEQ ID NO: 12, SEQ ID NO: 153, SEQ ID NO: 154, and SEQ ID NO: 155.
11. The oligonucleotide according to any one of embodiments 1-10, further comprising the sequence ATCCAGAGTGACGCAGCA (SEQ ID NO: 157) directly upstream of any one of the sequences SEQ ID NO: 1 to SEQ ID NO: 151, and the sequence TGGACACGGTGGCTTAGT (SEQ ID NO: 158) directly downstream of any one of the sequences SEQ ID NO: 1 to SEQ ID NO: 151.
12. The oligonucleotide according to embodiment 11, wherein the sequence is selected from a group consisting of SEQ ID NO: 159 to SEQ ID NO: 170.
13. The oligonucleotide according to any one of embodiments 1-12, which is single stranded.
14. The oligonucleotide according to any one of embodiments 1-13, which is an oligodeoxyribonucleotide.
15. The oligonucleotide according to any one of embodiments 1-14, which is an aptamer.
16. The oligonucleotide according to any one of embodiments 1-15, wherein the length of the oligonucleotide is 35-100 nucleotides.
17. The oligonucleotide according to the preceding embodiment, wherein the length of the oligonucleotide is 35-60 nucleotides.
18. The oligonucleotide according to any one of embodiments 1-17, comprising at least one modified oligonucleotide.
19. The oligonucleotide according to embodiment 18, wherein the modified oligonucleotide is selected from the group consisting of a deoxyribonucleotides, peptide nucleic acid (PNA), morpholino nucleic acid, glycol nucleic acid (GNA), threose nucleic acid (TNA), hexitol nucleic acids (HNA), a phosphorothioated backbone, and combinations thereof.
20. A conjugate comprising at least one delivery component and at least one cargo component, wherein the at least one delivery component comprises an oligonucleotide as defined in any one of embodiments 1-19 and the at least one cargo component comprises a deoxyribonucleotide sequence of more than 35 nucleotides.
21. A nucleic acid construct comprising at least one delivery component and at least one cargo component, wherein the at least one delivery component comprises an oligonucleotide as defined in any one of embodiments 1-19, and the at least one cargo component comprises a deoxyribonucleotide sequence of more than 35 nucleotides.
22. The nucleic acid construct according to embodiment 21, wherein the cargo component comprises a sequence of interest, in particular a gene of interest, optionally further comprising additional sequences that are required for correct gene expression.
23. The construct according to any one of embodiments 21-22, which is a closed linear DNA (cIDNA).
24. The cIDNA according to the preceding embodiment, comprising or consisting of a stem region comprising a double stranded gene of interest, wherein the stem region is covalently closed at both ends by hairpin loops, wherein the hairpin loops comprise the oligonucleotide as defined in any one of embodiments 1-19.
25. The cIDNA according to the preceding embodiment, wherein the stem region comprises an expression cassette.
26. The nucleic acid construct according to any one of embodiments 21-25, wherein the delivery component and the cargo component are separated by a spacer sequence.
27. The conjugate according to embodiment 20, or the nucleic acid construct according to any one of embodiments 21-26, comprising a functional molecule selected from the group consisting of a label, a metal nanoparticle, a polymer, a molecule bearing radioisotopes, a metal such as boron, or a liposome.
28. A method for delivering a cargo component to a cell comprising contacting a conjugate as defined in any one of embodiments 20 or 27, or a nucleic acid construct as defined in any one of embodiments 21-27, with the cell.
29. The method according to the preceding embodiment, wherein the delivery is in an *in vivo* environment.
30. The method according to any one of embodiments 28-29, wherein the delivery is to the cell nucleus.
31. A oligonucleotide according to any one of embodiments 1-19, a conjugate as defined in any one of embodiments 20-21 or 26-27, or a nucleic acid construct as defined in any one of embodiments 22-27, for use in therapy.
32. The oligonucleotide, conjugate or construct for use according to the preceding embodiment, wherein the therapy comprises delivery of a cargo component to a cell, wherein said cargo component comprises a deoxyribonucleotide sequence of more than 35 nucleotides.
33. The oligonucleotide, conjugate or nucleic acid construct for use according to the preceding embodiment, wherein the therapy is a DNA-based therapy, for example selected from gene therapy, gene-edition, cell-therapy (eg CAR-Ts), and vaccines.
34. The oligonucleotide, conjugate or nucleic acid construct for use according to embodiment 32, wherein the therapy is related to expression of a therapeutic protein.
35. Use of an oligonucleotide as defined in any one of embodiments 1-19, for delivering a cargo component to a cell, in particular, to the cell nucleus, wherein said cargo comprises a deoxyribonucleotide sequence of more than 35 nucleotides.
36. Use of an oligonucleotide as defined in any one of embodiments 1-19, for nuclear translocation of a cargo component, wherein said cargo component comprises a deoxyribonucleotide sequence of more than 35 nucleotides.
37. Use according to any one of the embodiments 35-36, wherein the delivery or nuclear translocation is in an *in vivo* environment.

Throughout the description and claims the word "comprise" and variations of the word, are not intended to exclude other technical features, additives, components, or steps. Furthermore, the word "comprise" encompasses the case of "consisting of" Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

### A. MATERIAL AND METHODS

### 1. Cell culture

HEK293T(ATCC) and JHH6 were cultured in DMEM and Williams medium (Life Technologies), respectively, supplemented with 10% inactivated FBS, 100 U/ml penicillin, 100 µg/ml streptomycin, and 100 mM sodium pyruvate. Transfections were performed with Lipofectamine 3000 (Life Technologies), following manufacturer's instructions.

Cell synchronization in HEK293T cells was carried out by the method of double tymidine shock. Cells were overnight incubated in the presence of 2 mM thymidine (Sigma) in two consecutive days, with a daily period without thymidine. After the second incubation period cells were synchronized in the S phase.

### 2. DNA aptamers targeting the nucleus

The DNA sequences, aptamer species, targeting the nucleus used in some examples (illustrated in Figures 2, 3, 4 and 5) have a sequence defined by SEQ ID Nos: 1 to 151 directly flanked by two constant sequences defined by SEQ ID NO: 157 and SEQ ID NO: 158 (5'-ATCCAGAGTGACGCAGCA-45N-TGGACACGGTGGCTTAGT-3'), for the detection of the aptamer by PCR amplification with specific primers (**Fig. 1A**). The aptamers were synthesized by Integrated DNA Technologies (IDT). The aptamers (200 pmol) were refolded by incubating at 95°C for 10 minutes and leaving to cool gradually until 37°C, and diluted in 3 mL of binding buffer (DPBS with 4.5 g/L glucose, 5 mM MgCl₂, 100 µg / mL yeast tRNA, and 1 mg/mL BSA). Cells were washed with washing buffer (DPBS with 4.5 g/L glucose, and 5 mM MgCl₂) and incubated with the aptamers in binding buffer for 1 hour. After this time, binding buffer was substituted for culture medium and incubated for 7 additional hours.

On the other hand, delivery of aptamers was also carried out by electroporation. A total of 5×10⁷ synchronized cells were resuspended in DPBS (1×10⁷ cells / mL) and electroporated with 20 pmol of aptamer pool by applying 500 V during 2 msec in a cell electroporator (BTX). DPBS is discarded after centrifugation (1200 rpm for 3 minutes) and cells were plated in culture medium and incubated for 8 hours.

Additionally, particular aptamers were ligated into a linear DNA encoding for the EGFP gene which contains Bsal restriction sites at the ends. Briefly, aptamers were amplified with primers that allowed the self-hybridization of 5' and 3' ends, building a Bsal restriction site. After amplification and strand separation, the aptamers were allowed to fold and restricted with the Bsal enzyme (New England Biolabs, R3733) for 6 hours. Bsal-restricted aptamers were ligated with Bsal-restricted linear DNA using a T4 DNA ligase (Promega, M179A) with overnight incubation at 16°C, and treated with exonuclease III, that efficiently degraded the non-ligated linear DNA or aptamers. Ligated product (1 pmol) was electroporated into 2.5×10⁷ synchronized HEK293T cells following the same protocol above described.

After cell incubation, nuclear fractionation was performed, and the aptamers were recovered by resuspension of nuclei in water and heating the suspension at 95°C for 10 minutes. After centrifugation at maximum speed for 5 minutes (4°C) supernatants were collected.

### 3. Nuclear fractionation

Cells were washed with cold DPBS, centrifuged at 500xg for 3 minutes (4°C), and cell pellets were resuspended in homogenization buffer (10 mM HEPES, 150 mM NaCl, 1 mM EDTA, 0.6 % NP-40, supplemented with protease inhibitors). Cell suspensions were passed 10 times through a 27-gauge needle in 1 mL syringe, incubated on ice for 5 minutes, and centrifuged at 200xg for 1 minute (4°C). Supernatants were transferred to a new tube and centrifuged at 500xg for 3 minutes (4°C). Supernatants were discarded and pellets containing nuclei were washed thrice with homogenization buffer.

### 4. PCR reactions and strand separation

PCR reactions were performed with a HS Taq DNA polymerase (Takara, R007A), with the following amplification conditions: an initial denaturation at 95°C for 3 minutes, cycles of denaturation at 95°C for 30 s, annealing at 56.3 °C for 30 s, and extension at 72°C for 30 s, and a final extension at 72°C for 3 minutes. Thus, double-stranded PCR product consisted in the forward strand corresponding with aptamer library and the biotin-labeled reverse strand. Separation of both strands was carried out by using streptavidin sepharose columns and eluting the forward strand with 200 nM NaOH. Finally, aptamers were purified in NAP-5 desalting columns, obtaining 1-mL aqueous.

### 5. HTS sequencing

Aptamers extracted from the nuclear compartment, were amplified by PCR and sequenced using the Illumina NGS platform after barcoding each PCR aptamer pool. Aptamers were demultiplexed and analyzed using a bioinformatic pipeline that quantifies each aptamer based on sequence identity.

### 6. Flow cytometry analysis of cellular uptake of fluorescent-labeled aptamers

Aptamers with SEQ ID Nos: 1-50, flanked by sequences defined by SEQ ID NO: 157 and SEQ ID NO: 158, were synthesized as ultramers (IDT) and labeled with Alexa Fluor 647 (AF647) at 5' end by PCR amplification with AF647-labeled forward (5'-ATCCAGAGTGACGCAGCA; SEQ ID NO: 175) and biotin-labeled reverse (5'-ACTAAGCCACCGTGTCCA; SEQ ID NO: 176) primers, and strand separation. HEK293T or JHH6 cells were incubated with AF647-labeled aptamers for 1 hour in DPBS and 5 additional hours in culture medium. After 6 hours cells were collected and resuspended in DPBS to measure AF647 fluorescence in a Cytoflex flow cytometer (Beckman).

### 7. Fluorescence microscopy analysis of aptamer nuclear uptake

HEK293T cells were incubated with AF647-labeled aptamers for 1 hour in DPBS and several additional hours in culture medium (only the first hour or overnight). The last hour cells were also treated with Hoescht for nuclear staining. After incubation, cells were visualized in an Operetta CLS High-Content Analysis System (Revvity) and images (4 fields/well, 6 z-stack/field) were automatically captured for both fluorescence (Hoescht and AF647). Quantification of fluorescence intensities were carried out with ImageJ software.

### 8. Construction of gapmer-aptamers

Gapmer-aptamers were synthesized from aptamers (ultramers, IDT) by amplification by PCR with the use of a forward primer that include the gapmer sequence (NICO7.1 with SEQ ID NO: 171 or NEAT1-1 with SEQ ID NO: 172) at the 5' position and the reverse primer labelled with biotin (see figure 4B). To avoid the synthesis of the complementary strand to the gapmer part we included a 2'-o-methyl base in the forward primer. PCR reactions were performed with a HS Taq DNA polymerase (Takara, R007A), as described above. The strand containing the gapmer was isolated by using streptavidin sepharose columns and eluting with 200 nM NaOH. Gapmer aptamers were purified in NAP-5 desalting columns, obtaining 1-mL aqueous solutions.

### 9. Split-Nanoluc reporter system for gapmer-aptamers

The Nanoluc luciferase can be separated in two subunits: a large subunit called "Large BIT" (LgBIT) of 18 kDa, and a small domain called "High BIT" (HiBIT) of 1.3 KDa and 11 aminoacids. We cloned the HiBIT and the LgBIT sequences together with the EGFP sequence in frame into the pcDNA 3.1 plasmid. The HiBIT and the LgBIT sequences were separated by an intron that contained the target sequences for gapmers against NIHCOLE (GenelD 107986389) and NEAT1 (GenelD 283131) IncRNAs. These sequences were also included in the 3'-untranslated region. Controls lacking one or both targets were also constructed.

HEK293T cells were transfected with these constructs and gapmer-aptamers and luminescence was measured 24 hours later using the NanoGlo luciferase assay system (Promega).

### 10. Tornado-Broccoli reporter system for gapmer-aptamers

The fluorogenic RNA called Broccoli becomes fluorescent under incubation with the DFHBI compound. It has been engineered within the Tornado-circularizing system to generate a stable circular RNA. We added to this construct an accessory part connected with a F30 stem moiety that contains the target sequences for gapmers against NIHCOLE IncRNA. The whole construct was cloned into the Sleeping Beauty plasmid, which also expresses mCherry and a puromycin resistance gene (that we called pSB/Tornado-Broccoli-targets).

Stable HEK293T cells were established by co-transfection of the pSB/Tornado-Broccoli-targets with the Sleeping Beauty transposase, and selection with puromycin. Finally, clones were obtained and selected according with the fluorescent signal in the presence of DFHBI-1T (Tocris). The same process was carried out with the original Tornado-Broccoli plasmid (Addgene) to have a control without gapmer targets.

HEK293T/Tornado-Broccoli-target stable cells were incubated with gapmer- aptamers for 1 hour in DPBS and 23 additional hours in culture medium. After incubation, cells were collected and resuspended in DPBS to measure AF647 fluorescence in a Cytoflex flow cytometer (Beckman).

### 11. Flow cytometry analysis of nuclear delivery of aptamer-closed oDNAs

Linear DNA encoding EGFP gene with individual aptamers (1-12, 1FV, 2FV and 3FN) closing the ends (oDNAs) were synthesized (see figure 1B). The individual aptamers were those with SEQ ID NO: 159 to SEQ ID NO: 170 (comprising SEQ ID NO: 1 to SEQ ID NO:12 flanked by SEQ ID NO: 157 and SEQ ID NO: 158 and additional linker sequences) and SEQ ID NO: 153, SEQ ID NO: 154 and SEQ ID NO: 155.

For preparing the oDNAs 1-12, 1FV, 2FV and 3FN, SEQ ID NO:173 comprising EGFP (figure 1C) was digested with Bsal, whereby the underlined and in italics nucleotides at 5' are removed while bolded nucleotides "CCCT" are kept (as illustrated in Figure 1E). At the same time, the underlined and in italics nucleotides at 3' are removed while bolded nucleotides are kept (as illustrated in Figure 1F). The digested sequence was then ligated with the aptamers. Thus, during ligation process, oligonucleotides bearing "AGGG" sequence (SEQ ID 153 to 155 and 159 to 170) ligate at 5' position as well as at 3' position leading to corresponding oDNA where adaptors (those oligonucleotides SEQ ID 153 to 155 and 159 to 170) are the corresponding close-ends (labeled as "ssDNA aptamer" in Figure 1B) of these linear DNA sequences: the oDNAs. These oDNA(s) are produced and ready for testing; results are shown in Figures 6 and 7.

**Table 1. Adaptors for preparing oDNAs (ssDNA aptamers). For each ssDNA: bolded nucleotides correspond to the aptamers 1-12 (SEQ ID NO: 1 to 12 respectively; eg oDNA1 bearing SEQ ID NO:1 and so on); underlined nucleotides correspond to SEQ ID NO: 157 and SEQ ID NO: 158 and the rest of nucleotides correspond to additional linker sequences to fit example requirements (eg In this case, using Bsal as restriction enzyme).**

| **oDNA name** | **Adaptor sequences for preparing oDNA (ssDNA aptamers)** | **SEQ ID No. for the ssDNA aptamer** |
|---|---|---|
| oDNA 1 | AGGGAGAGAGAAAATCCAGAGTGACGCAGCACCGCCTGTGTCTTACCT AATGTTGCGTCATCTCTGTGCGTCACGATGGACACGGTGGCTTAGTAA AATCTCTCT | SEQ ID NO: 159 |
| oDNA 2 | AGGGAGAGAGAAAATCCAGAGTGACGCAGCAGCACACCACATCATACT GTGTCCAACCGTTTTGCGAGTCCTCCCGTGGACACGGTGGCTTAGTAA AATCTCTCT | SEQ ID NO: 160 |
| oDNA 3 | AGGGAGAGAGAAAATCCAGAGTGACGCAGCATGGTGTGTGTGCTTATC CCTTTCTTTGGTCGGTTGCTGCGTTTTGTGGACACGGTGGCTTAGTAAA ATCTCTCT | SEQ ID NO: 161 |
| oDNA 4 | AGGGAGAGAGAAAATCCAGAGTGACGCAGCACAAACCACGACATACC TTTTCCGTTCCAGGCAGGTCTTTATTCCGTGGACACGGTGGCTTAGTAA AATCTCTCT | SEQ ID NO: 162 |
| oDNA 5 | AGGGAGAGAGAAAATCCAGAGTGACGCAGCACCCCACCGCTCACCAT AGTCACTCTCATTGTCCAACCTCTCACTCTGGACACGGTGGCTTAGTAA AATCTCTCT | SEQ ID NO: 163 |
| oDNA 6 | AGGGAGAGAGAAAATCCAGAGTGACGCAGCACCACACATCCAACCGT AAATGCTCCTTGTCCAGCAACCCTTACCGTGGACACGGTGGCTTAGTA AAATCTCTCT | SEQ ID NO: 164 |
| oDNA 7 | AGGGAGAGAGAAAATCCAGAGTGACGCAGCACAACACACATTTCCTCC CCCGGCACTGCTGGTCGATTCGCGCGGTTGGACACGGTGGCTTAGTAA AATCTCTCT | SEQ ID NO: 165 |
| oDNA 8 | AGGGAGAGAGAAAATCCAGAGTGACGCAGCAGGTTATGTGGGTGGTG CTGCTGTGATGGTGATGCGTCCGATGGGGTGGACACGGTGGCTTAGTA AAATCTCTCT | SEQ ID NO: 166 |
| oDNA 9 | AGGGAGAGAGAAAATCCAGAGTGACGCAGCACACAACTGAACACATTG CTGCACGTCTTGATCCCTAGCTGGTTGCTGGACACGGTGGCTTAGTAA AATCTCTCT | SEQ ID NO: 167 |
| oDNA 10 | AGGGAGAGAGAAAATCCAGAGTGACGCAGCAACAACGCCTCTCTTCTT CTTGCCCTGAGAACACACACACTACCCGTGGACACGGTGGCTTAGTAA AATCTCTCT | SEQ ID NO: 168 |
| oDNA 11 | AGGGAGAGAGAAAATCCAGAGTGACGCAGCAACACACACCATGCTTCT | SEQ ID NO: 169 |
| | TTGCCCTTTCTCTCTTTTGCTTCCCCTGTGGACACGGTGGCTTAGTAAA ATCTCTCT | |
| oDNA 12 | AGGGAGAGAGAAAATCCAGAGTGACGCAGCAACAACAATTACCCGTAG TCCAGCATCCGACGTTCTCCTGCCCTCGTGGACACGGTGGCTTAGTAA AATCTCTCT | SEQ ID NO: 170 |
| oDNA 1FV | AGGGCCAGGCTCCCCAGCAGGCAGAAGTATGCAAAGCATGCATCTCAA TTAGTCAGCAACCAATCCACTCAGGATTGGTTGCTGACTAATTGAGATG CATGCTTTGCATACTTCTGCCTGCTGGGGAGCCTGG | SEQ ID NO: 153 |
| oDNA 2FV | AGGGCCAGGCTCCCCAGCAGGCAGAAGTATGCAAAGCATGCATCTCAA TTAGTCAGCAACCACATCCATTAACTCATAATGGATGTGGTTGCTGACTA ATTGAGATGCATGCTTTGCATACTTCTGCCTGCTGGGGAGCCTGG | SEQ ID NO: 154 |
| oDNA 3FN | AGGGCTGGGGACTTTCCAGCCTGGGGACTTTCCAGCTGGGACTTTCCA GGATCCACTCAGGATCCTGGAAAGTCCCAGCTGGAAAGTCCCCAGGCT GGAAAGTCCCCAG | SEQ ID NO: 155 |

For preparing the control oDNA (herein termed IcDNA), SEQ ID NO:174 comprising EGFP as well as target sequences for protelomerase (TelN) at 5' and 3' (in bold), as depicted in figure 1G (as well as in Figures I and K), was digested with TeIN, leading to cleavage and joining of those specific sequences in bold. Thus, once TelN is utilized, this process leads to corresponding control IcDNA which bears the Gol (coding for EGFP) as well as specific sequences (nucleotides of protelomerase target sequence) as close-ends, as shown in reported below (Figures 1J and 1L) but no aptamers or any other sequence.

HEK293 cells were incubated with 300 ng of oDNAs molecules or a linear DNA closed with a small oligonucleotide (linear closed DNA or IcDNA) for 24 hours (first hour in DPBS and the rest with culture medium). Then, cells were collected, resuspended in DPBS and fluorescence for EGFP analyzed in a Cytoflex flow cytometer (Beckman). On the other hand, HEK293 cells were transfected with 300 ng of oDNAs or IcDNA by JetPEI transfection reagent (Polyplus), and after 6, 12 and 24 hours analyzed by flow cytometry as described above. EGFP fluorescence was also measured in a plate fluorimeter (GloMax, Promega) in a fraction of cell suspensions.

### RESULTS

### 1. DNA aptamers that internalize into eukaryotic cells

To assess if the DNA aptamers of the invention had the capacity to be translocated from the extracellular medium to the interior of eukaryotic cells, HEK293T cells were incubated for 1 hour with the ssDNA aptamer pool in DPBS supplemented with 4.5 g/L glucose, 5 mM MgCl₂, 100 µg / mL yeast tRNA, and 1 mg/mL BSA. DPBS was replaced by culture medium and cells were incubated for 7 additional hours. At the end, we fractionated the nucleus, and amplified and sequenced the aptamers contained in therein. It was found that the aptamers of SEQ ID Nos: 1-151 were found with high abundance in the nucleus.

Additional experiments were carried out to confirm that the aptamers were able to translocate from the cytoplasm to the nucleus. To this end, the aptamers were electroporated into synchronized HEK293T cells. Eight hours later, we fractionated the nucleus and purified the aptamers. After amplification and sequencing it was again was found that the aptamers were present with high abundance in the nucleus.

Finally, to find candidates able to translocate a large cargo from the cytoplasm to the nucleus, the aptamers were ligated to a linear DNA expressing GFP to form an oDNA **(****Fig. 1B-C****)** and electroporated into synchronized HEK293T cells. After electroporation, cells were incubated for 8 hours before nuclear fractionation and recovery of aptamer containing sequences. After amplification and sequencing it was again found that the aptamers were present with high abundance in the nucleus.

### 2. DNA aptamers are internalized by free uptake

Aptamers of SEQ ID Nos: 1 to 50 flanked by the two constant sequences as defined above were synthesized as ultramers (IDT Technologies). PCR amplification with Alexa Fluor 647 (AF647)-labelled forward primer and biotinylated reverse primer and strand separation was used to bind all oligos to AF647 fluorescent moiety (**Fig. 2A**). Then, we incubated HEK293T cells for 6 hours with the 50 labeled aptamers and investigated their cell uptake by measuring fluorescence by flow cytometry. All the cells incubated with AF647-labeled aptamers showed higher fluorescence than HEK293T control cells, indicating that all of them are properly internalized into cells. Aptamers with SEQ ID Nos: 6, 7, 10, 11, 14, 19, 21, 23 and 24 showed a particularly increased uptake. Interestingly, similar results were obtained with the hepatocellular carcinoma cell line JHH6, suggesting that aptamers may be using conserved mechanisms shared by different cells. As an example, **Fig. 2B** shows the difference in fluorescent signal between the HEK293T cells incubated with a reference control aptamer and the aptamer 24.

We also studied the pattern of aptamers 11, 14, 19, 21 and 24 following cellular uptake by means of fluorescence microscopy at different time points. Interestingly, compared to negative controls, all aptamers present binding to the cell surface 1h after incubation with HEK293T cells, as shown for aptamer 24 in the **Fig 3A****,** and nuclear staining after further incubation. Noteworthy, following an overnight incubation for 16 hours, aptamer 24 shows the highest signal with foci in the cytoplasm and a more diffuse staining in the nucleus **(****Fig. 3B****).** Quantification of AF647 fluorescent signal co-localized with nuclear Hoescht signal confirmed that aptamer 24 showed the highest fluorescence intensity after the overnight incubation (**Fig. 3C**).

### 3. Fusion of aptamer 24 with gapmers leads to RNA inhibition in reporter systems

In order to investigate whether aptamers would help for antisense oligonucleotide (ASO) internalization and functionality, we selected aptamer 24. First, we evaluated if a fusion aptamer 24-ASO may affect ASO functionality by steric hindrance. For this, we built a construct encoding for split Nanoluc. Nanoluc HiBIT and LgBIT were separated by an intron which contains the target sequences for specific ASOs against NEAT1 and NIHCOLE IncRNAs **(****Fig. 4A****).** Then, we fused aptamer 24 with an ASO with a scrambled sequence or ASOs targeting NEAT1 or NIHCOLE. This was achieved by amplifying the aptamer with forward primers containing the gapmer and a biotin-labeled reverse primer which allowed strand separation **(****Fig. 4B****).** Co-transfection of the Split-Nanoluc plasmid and NEAT1 or NIHCOLE ASO-aptamer 24 fusions reduced luminescence compared to control ASO-aptamer (**Figure 4C**), indicating that the specific ASOs did not lose functionality when coupled to the aptamer. As expected, inhibition was highest when more ASO-aptamer was used.

Finally, to evaluate whether the aptamer was capable of driving the ASO within the cell, we used HEK293T cells stably expressing a Tornado-Broccoli-NIHCOLE target sequence **(****Fig. 5A****).** These cells express the Broccoli fluorogenic RNA in a circular molecule (Tornado system) and produce green fluorescence when they are treated with the substrate DFHBI-1T. Incubation of HEK293T/Tornado-Broccoli-target with the ASO-aptamer 24 reduced cell fluorescence compared to that of cells incubated with the control ASO-aptamer 24 (**Figure 5B**). This indicates that aptamer 24 translocated a functional ASO into the cell.

### 4. Some DNA aptamers fused to GFP reporter oDNAs enhance their translocation to the nucleus

ssDNA aptamers 1-12 (SEQ ID NO: 159 to SEQ ID NO: 170), 1FV (SEQ ID NO: 153), 2FV (SEQ ID NO: 154) and 3FN (SEQ ID NO: 155) were ligated to a large DNA molecule (more than 2 kb ). We synthesized linear closed dsDNA encoding EGFP gene with individual ssDNA aptamers closing the ends (oDNAs, **Fig. 1B-F**), resulting in oDNAs 1 to 12, oDNA 1FV, oDNA 2FV and oDNA 3FN. As control, we employed a linear closed DNA,or IcDNA, also encoding EGFP gene, where closed ends are specific sequences (**Figures 1J and 1L****,** for 5' and 3' respectively)..

We first evaluated the capacity of the oDNA to translocate from the culture media to the cell nucleus. Thus, HEK293 cells were incubated in the presence of the oDNAs or IcDNA for 1 day and GFP expression was evaluated. The results show that control cells, cells incubated with IcDNA, show GFP levels lower than 5%, considered as background. Incubation with oDNAs 1, 2, 3, and 4 resulted in substantially increased number of GFP expressing cells when compared to control IcDNA (**Fig. 6A**). Best results were obtained with oDNA 4, whose incubation resulted in GFP levels in around 15% of the cells. Evaluation of GFP fluorescence by FACS indicates that incubation with oDNA 4 results in most cells slightly greener than with the control IcDNA and a subpopulation of cells with higher GFP levels (**Fig. 6B**). Our results indicate that aptamers 1, 2, 3, and 4 are capable of a more efficient internalization of oDNAs from the cell media.

To determine whether the selected aptamers can also aid nuclear internalization from the cytoplasm, oDNAs 4 to 12, oDNA 1FV, oDNA 2FV and oDNA 3FN, were transfected with JetPEI reagent (Polyplus) into HEK293 cells and compared with the control IcDNA. We expected that oDNA would get to the nucleus more efficiently and therefore, show increased cell fluorescence than the control. We tested by flow cytometry the fluorescence level in cells transfected with oDNAs after 6, 12 and 24 hours. One day after transfection most cells (> 95% of total cells) showed green fluorescence, while 6h after transfection most cells had only a slight increase in fluorescence compared to untransfected cells (data not shown). Interestingly, at 12 hours after transfection cells showed a significant level of fluorescence compared with untransfected cells but reduced compared to that observed at one day after transfection. At this time point, the percentage of EGFP positive cells, fluorescence observed in transfected cells, was clearly lower in the control IcDNA than in those assays transfected with the oDNAs. (**Fig. 7A**. Evaluation of the EGFP fluorescence levels with the oDNA clearly shows a superior response than using IcDNA (**Fig. 7B**). The oDNA(s) bearing selected aptamers as adaptors (close-ends) also provided a clear increase of GFP fluorescence in the whole cell population as well as an increase in the number of cells with a high level of EGFP (**Fig. 7C****).** Altogether, these oDNA candidates allowed an increase of GFP fluorescence in the whole cell population as well as an increase in the number of cells with a high level of EGFP in comparison with IcDNA.

### Citation List

WO2021/152146
WO 2021/152147
Karlin and Altschul (1990) Proc. Natl. Acad. Sci. USA 87:2264-68
Karlin and Altschul (1993) Proc. Natl. Acad. Sci. USA 90:5873-77
Altschul, et al. (1990) J. Mol. Biol. 215:403-10
Altschul et al. (1997) Nucleic Acids Res. 25(17):3389-3402

## Claims

1. An oligonucleotide comprising or consisting of:
a sequence selected from the group consisting of SEQ ID NO: 1 to SEQ ID NO: 156, or
a sequence having at least 80% identity with a sequence selected from the group consisting of SEQ ID NO: 1 to SEQ ID NO: 156 and is capable of translocating to the cell nucleus.

2. The oligonucleotide according to claim 1, comprising or consisting of:
a sequence selected from the group consisting of SEQ ID NO: 1 to SEQ ID NO: 50, in particular from SEQ ID NO: 1 to SEQ ID NO: 25, more in particular from SEQ ID NO: 1 to SEQ ID NO: 12, or
a sequence having at least 80% identity with a sequence selected from the group consisting of SEQ ID NO: 1 to SEQ ID NO: 50, in particular from SEQ ID NO: 1 to SEQ ID NO: 25, more in particular from SEQ ID NO: 1 to SEQ ID NO: 12, and is capable of translocating to the cell nucleus.

3. The oligonucleotide according to claim 2, comprising or consisting of:
a sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 8, and SEQ ID NO: 12,or
a sequence having at least 80% identity with a sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 8, and SEQ ID NO: 12, and is capable of translocating to the cell nucleus.

4. The oligonucleotide according to any one of claims 1-3, further comprising the sequence ATCCAGAGTGACGCAGCA (SEQ ID NO: 157) directly upstream from the sequence selected from SEQ ID NO: 1 to SEQ ID NO: 151, and the sequence TGGACACGGTGGCTTAGT (SEQ ID NO: 158) directly downstream from the sequence selected from SEQ ID NO: 1 to SEQ ID NO: 151.

5. The oligonucleotide according to claims 1-4, comprising or consisting of a sequence selected from the group consisting of SEQ ID NO: 159 to SEQ ID NO: 170, or a sequence having at least 80% identity with a sequence selected from the group consisting of SEQ ID NO: 159 to SEQ ID NO: 170 and capable of translocating to the cell nucleus.

6. The oligonucleotide according to claim 1, comprising or consisting of:
a sequence selected from the group consisting of SEQ ID NO: 152 to SEQ ID NO: 156, in particular selected from SEQ ID NO: 153, SEQ ID NO: 154, and SEQ ID NO: 155, or
a sequence having at least 80% identity with a sequence selected from the group consisting of SEQ ID NO: 152 to SEQ ID NO: 156, in particular selected from SEQ ID NO: 153, SEQ ID NO: 154, and SEQ ID NO: 155, and is capable of translocating to the cell nucleus.

7. The oligonucleotide according to any one of claims 1-6, which is single stranded and, optionally, an oligodeoxyribonucleotide, preferably comprising modified nucleotides.

8. A nucleic acid construct comprising at least one delivery component and at least one cargo component, wherein the delivery component comprises an oligonucleotide as defined in any one of claims 1-7 and the at least one cargo component is a deoxyribonucleotide sequence of more than 35 nucleotides.

9. The nucleic acid construct according to claim 8, wherein the cargo component comprises a sequence of interest, in particular a gene of interest, optionally further comprising additional sequences that are required for correct gene expression.

10. The construct according to any one of claims 8-9, which is a closed linear DNA (cIDNA).

11. The cIDNA according to the preceding claim, consisting of a stem region comprising a double stranded gene of interest, wherein the stem region is covalently closed at both ends by hairpin loops, wherein the hairpin loops comprise the oligonucleotide as defined in any one of claims 1-7.

12. A method for delivering a cargo component to a cell, in particular for delivering a cargo component to a cell in an *in vivo* environment, said method comprising contacting a construct as defined in any one of claims 8-11 with the cell.

13. An oligonucleotide according to any one of claims 1-7, or a construct according to any one of claims 8-11, for use in therapy, in particular for use in a DNA-based therapy, for example selected from gene therapy, gene-edition, cell-therapy (eg CAR-Ts), vaccines, and expression of a therapeutic protein.

14. The oligonucleotide or construct for use according to claim 13, wherein the therapy comprises delivery of a cargo component to a cell, wherein the cargo component is a deoxyribonucleotide sequence of more than 35 nucleotides.

15. Use of an oligonucleotide as defined in any one of claims 1-7, for delivering a cargo component to a cell, in particular, to the cell nucleus, more in particular, in an *in vivo* environment, wherein the cargo component is a deoxyribonucleotide sequence of more than 35 nucleotides.
